# EUROPÄISCHE PATENTSCHRIFT

(11) **EP 3 426 322 B1**
(45) Veröffentlichungstag und Bekanntmachung des Hinweises auf die Patenterteilung: **28.08.2024**
(21) Anmeldenummer: 17709578.3
(22) Anmeldetag: 14.02.2017
(51) Int. Cl.: A61M 1/30, A61M 1/32

(54) **SYSTEM ZUR EXTRAKORPORALEN ELIMINATION VON KOHLENMONOXID**
SYSTEM FOR THE EXTRACORPOREAL ELIMINATION OF CARBON MONOXIDE
SYSTÈME D'ÉPURATION EXTRACORPORELLE DU MONOXYDE DE CARBONE

(30) Priorität: 11.03.2016 DE 102016002950
(43) Veröffentlichungstag der Anmeldung: 16.01.2019
(73) Patentinhaber: Rheinisch-Westfälische Technische Hochschule Aachen, 52062 Aachen (DE)
(72) Erfinder: ARENS, Jutta, 52072 Aachen (DE); BORCHARDT, Ralf, 52066 Aachen (DE); SCHLANSTEIN, Peter, Christian, 52070 Aachen (DE); SCHMITZ-RODE, Thomas, 52070 Aachen (DE); SCHRAVEN, Lotte, 52074 Aachen (DE); WAGNER, Georg, 52066 Aachen (DE); STEINSEIFER, Ulrich, 4730 Hauset (BE)
(74) Vertreter: Cohausz Hannig Borkowski Wißgott
(86) Internationale Anmeldenummer: PCT/EP2017/000202
(87) Internationale Veröffentlichungsnummer: WO 2017/153034

(56) Entgegenhaltungen:
- EP-A2- 0 240 101
- WO-A1-2005/028002
- DE-A1- 3 232 716
- US-A- 5 643 215
- US-A1- 2012 172 781

## Beschreibung

Die Erfindung betrifft ein System zur extrakorporalen Elimination von Kohlenmonoxid, umfassend zumindest eine Pumpe und eine Gasaustauschkammer, wobei wenigstens eine Pumpe durch einen mit einer Kanüle verbindbaren ersten Schlauchabschnitt mit dem Blutkreislauf einer Person verbindbar ist und über einen zweiten Schlauchabschnitt mit einer Gasaustauschkammer verbunden ist.

Das Hämoglobin im Blut steht für die Bindung von Sauerstoff (O₂) zu Verfügung, wobei Sauerstoff an die Eisenatome im Hämoglobin gebunden wird, so dass der Sauerstoff durch den Blutstrom von der Lunge in andere Organe und Gewebe transportiert wird. Ähnlich der SauerstoffBindung an Hämoglobin bindet auch Kohlenmonoxid (CO) an Hämoglobin, wobei dies mit einer bis zu 300-fach höheren Affinität als beim Sauerstoff stattfindet. Ist ein Eisenatom durch ein Kohlenmonoxidmolekül "blockiert", steht es für eine Bindung eines Sauerstoffmoleküls nicht mehr zur Verfügung. Durch die Blockade des Hämoglobins durch die Bindung mit dem Kohlenmonoxid, wird die Aufnahme- und Transportfähigkeit des Blutes für Sauerstoff herabgesetzt und dem Patient droht die innere Erstickung. Zudem gelangt Kohlenmonoxid mit dem Blut in den Körperkreislauf und lagert sich in Organen ab, beispielsweise im Herzmuskelgewebe, wo es gebunden an Myoglobin zur Unterversorgung des Herzens mit Sauerstoff und zum Herzinfarkt führen kann.

Bei einer leichten Kohlenmonoxidvergiftung kann eine medikamentöse Therapie und/oder eine Therapie mit Sauerstoffbeatmung eingeleitet werden. Schwere Kohlenstoffmonoxidvergiftungen erfordern allerdings zur effektiven Therapie eine Behandlung des Patienten in einer Druckkammer mit hyperbarer Oxygenierung (HBO). Im Rahmen einer HBO-Therapie wird der Patient in einer Druckkammer platziert, er atmet Sauerstoff über eine Maske und der Umgebungsdruck in der Kammer wird erhöht. Durch den erhöhten Sauerstoffpartialdruck bzw. den physikalisch gelösten Sauerstoffanteil im Blut, der durch eine Einatmung von reinem Sauerstoff und dem erhöhten Umgebungsdruck entsteht, wird das Kohlenmonoxid deutlich schneller aus dem Blut eliminiert.

Die Elimination von Kohlenmonoxid mittels einer HBO-Therapie ist ein verhältnismäßig langsamer Prozess, der über die Dauer von Minuten bis Stunden durchzuführen ist. Zudem ist die Anzahl an HBO-Zentren beschränkt. Beispielsweise gibt es in Deutschland 25 HBO-Zentren, die bundesweit räumlich ungleich verteilt sind, sodass eine schnelle und flächendeckende HBO-Therapie bei Kohlenmonoxidvergiftungen nicht gewährleistet ist. Eine schnelle Behandlung von Kohlenmonoxidvergiftungen ist jedoch wichtig, um die Sauerstoffversorgung des Patienten wiederherzustellen und eine Verbreitung des Kohlenmonoxids zu minimieren. Auf diese Weise sind schwere Folgeschäden oder ein tödlicher Verlauf durch eine Sauerstoffunterversorgung verhinderbar.

Ein Verfahren und ein System zur Behandlung einer Kohlenmonoxidvergiftung wird im Patentdokument WO 2012/082257 A1 vorgestellt. Das mit Kohlenmonoxid beladenem Hämoglobin (COHB) wird aus dem Blutkreislauf einer Person mittels einer Pumpe in eine Filtereinheit befördert. In der Filtereinheit wird das Kohlenmonoxid durch Sauerstoff ersetzt und über einen zweiten Schlauch in den Blutkreislauf des Patienten zugeführt. Die Filtereinheit ist dabei so aufgebaut, dass das Blut und ein Entnahmemedium, durch eine gasdurchlässige Membran getrennt, tangential aneinander vorbeifließen. Durch die gasdurchlässige Membran findet ein Gasaustausch zwischen dem mit Sauerstoff angereicherten Entnahmemedium und dem mit Kohlenstoffmonoxid belegten Hämoglobin statt. Somit ist kein direkter Kontakt zwischen Blut und Entnahmemedium möglich, was einen zusätzlichen Widerstand für einen effektiven Gasaustausch darstellt. Die Filtereinheit wird unidirektional durchströmt, somit kann mit dem vorgestellten System nur ein kontinuierlicher Prozess durchgeführt werden, bei dem während der Behandlung das mit Kohlenmonoxid beladene Blut über eine erste Kanüle aus dem Körper geleitet wird und durch eine zweite Kanüle sauerstoffangereichertes Blut in den Blutkreislauf zurückgeführt wird. Somit ist es bei diesem Verfahren notwendig zwei Kanülen am Patienten anzulegen. Des Weiteren ist ein zweiter Kreislauf mit einer zusätzlichen Pumpe für das Entnahmemedium notwendig, so dass die Komplexität dieses Aufbaus erhöht ist.

Aus US 5,643,215 ist ein Blasen-Oxygenator bekannt, der mit nur einer Kanüle an den Blutkreislauf des Patienten angeschlossen wird.

Die Aufgabe der vorliegenden Erfindung besteht darin, ein einfaches System zur extrakorporalen Elimination von Kohlenmonoxid bereitzustellen, das einen geringen Wartungsaufwand aufweist, transportabel nutzbar ist, minimal invasiv an den Blutkreislauf einer Person anschließbar ist und mit einfachsten Mitteln zu betreiben ist.

Erfindungsgemäß wird diese Aufgabe durch ein System mit den technischen Merkmalen des Anspruchs 1 gelöst, wobei das System zur extrakorporalen Elimination von Kohlenmonoxid dadurch gekennzeichnet ist, dass es dazu eingerichtet ist mittels zumindest einer Pumpe über einen ersten Schlauchabschnitt aus dem Blutkreislauf einer Person Blut, insbesondere über den zweiten Schlauchabschnitt, in eine Gasaustauschkammer abzuführen und aus der Gasaustauschkammer über denselben ersten Schlauchabschnitt in den Blutkreislauf der Person zurückzuführen.

Dadurch, dass bei diesem System das Blut über den ersten Schlauchabschnitt entnommen und auch über denselben ersten Schlauchabschnitt in den Körper zurückgeführt wird, ist es möglich eine extrakorporale Elimination von Kohlenmonoxid durch das Anlegen eines einzigen Zugangs zu realisieren. Das System ermöglicht über den ersten Schlauchabschnitt somit eine portionsweise Behandlung von mit Kohlenmonoxid beladenem Hämoglobin in der Gasaustauschkammer. Erster Schlauchabschnitt und zweiter Schlauchabschnitt können dabei bevorzugt durch denselben ununterbrochenen Schlauch gebildet werden.

Vorteilhafterweise wird dadurch eine minimal invasive Lösung für die extrakorporale Elimination von Kohlenmonoxid realisiert, da anstatt zwei Zugängen nur noch ein einzelner Zugang zum Blutkreislauf der Person notwendig ist. Zudem wird die Zeit die notwendig ist, um das System in Betriebsbereitschaft zu versetzen signifikant reduziert, da das Anlegen eines zweiten Zugangs entfällt. Zusätzlich ist das Füllvolumen des Systems geringer, weil kein zweiter Zugang mit Schlauchsystem für den Betrieb mit Blut gefüllt werden muss.

Eine vorteilhafte Ausführung der Erfindung sieht es vor, dass das System eine Pumpe aufweist, die bidirektional betreibbar ist.

Auf diese Weise ist es möglich das Blut über nur einen einzigen Schlauch und mittels nur einer Pumpe aus dem Blutkreislauf der Person in die Gasaustauschkammer abzuführen und aus der Gasaustauschkammer in den Blutkreislauf zurückzuführen. Beim Betrieb der bidirektional betreibbaren Pumpe in einer ersten Pumprichtung bzw. Drehrichtung wird das Blut also aus dem Blutkreislauf der Person in eine Gasaustauschkammer abgeführt. Bei umgekehrter Drehrichtung wird das mit Sauerstoff angereicherte Blut wieder in den Blutkreislauf der Person zurückgeführt.

Die Verwendung einer bidirektionalen Pumpe hat den Vorteil, dass für eine portionsweise Behandlung des Blutes in der Gasaustauschkammer nur eine Pumpe notwendig ist, was zu einem möglichst einfachen Aufbau führt, einfach zu steuern und günstig zu realisieren ist. Zudem wird durch die Verwendung nur einer bidirektionalen Pumpe Platz und Gewicht gespart, so dass vorteilhafterweise ein transportables System zur extrakorporalen Elimination von Kohlenmonoxid zu verwirklichbar ist.

Eine weitere Ausführung der Erfindung sieht es vor, dass der erste Schlauchabschnitt, der über eine Kanüle mit dem Blutkreislauf der Person verbindbar ist, mit zwei unidirektional betreibbaren Pumpen verbunden ist. Der erste Schlauchabschnitt wird vorzugsweise mittels eines Y-Konnektors aufgeteilt und über diesen mit den beiden unidirektional betreibbaren Pumpen verbunden. Durch die Verwendung von zwei unidirektional betreibbaren Pumpen ist es möglich das Blut portionsweise in der Gasaustauschkammer zu behandeln. Dafür wird beispielsweise eine erste Pumpe aktiviert, um das Blut aus dem Blutkreislauf der Person in die Gasaustauschkammer zu führen. Eine zweite unidirektionale Pumpe wird aktiviert, um das Blut aus der Gasaustauschkammer in den Blutkreislauf der Person zurückzuführen. Bezogen auf den ersten Schlauchabschnitt fördern somit beide Pumpen in entgegengesetzten Richtungen. Die Verwendung von zwei unidirektionalen Pumpen hat den Vorteil, dass man vergleichsweise günstige Pumpen für den Aufbau des erfindungsgemäßen Systems verwenden kann, die zudem eine hohe Zuverlässigkeit aufweisen.

Eine besonders bevorzugte weitere Ausführungsform der Erfindung sieht es vor, dass die Pumpe oder die Pumpen Schlauchpumpen sind.

Das zu befördernde Medium wird dabei durch äußere mechanische Verformung eines Schlauches durch diesen hindurch gedrückt. Durch die Verwendung einer Schlauchpumpe kann somit das Blut über einen Schlauch aus dem Blutkreislauf der Person abgeführt und wieder zurückgeführt werden ohne mit anderen Komponenten der Pumpe in Kontakt zu kommen.

Dies bietet den Vorteil, dass die Pumpe selber bei der Umstellung auf neue Patienten nicht gereinigt werden muss, sondern nur der Austausch des Schlauches notwendig ist um einen Austausch von Blut unterschiedlicher Patienten zu verhindern. Durch die Verwendung von Schlauchpumpen ist deshalb eine hygienische Realisierung des Systems zur extrakorporalen Elimination von Kohlenmonoxid möglich.

Die Erfindung sieht weiterhin vor, dass durch die Pumpe oder die Pumpen im deaktivierten nichtpumpenden Zustand ein Fluss zwischen dem ersten Schlauchabschnitt und dem zweiten Schlauchabschnitt verhindert ist.

Der erste Schlauchabschnitt ist dabei mit einer Kanüle verbindbar wohingegen der zweite Schlauchabschnitt mit der Gasaustauschkammer verbunden ist.

Beispielsweise lässt sich der Fluss im deaktivierten Zustand bei der Verwendung einer Schlauchpumpe verhindern, da die mechanische Verformung des Schlauches von außen dazu führt, dass ein Fluid diesen Schlauch nicht passieren kann. Durch die Verwendung einer Pumpe, die im deaktivierten Zustand einen Fluss zwischen dem ersten Schlauchabschnitt bzw. dem Blutkreislauf einer Person und dem zweiten Schlauchabschnitt bzw. der Gasaustauschkammer verhindert, ist es möglich eine Trennung zwischen dem Blutkreislauf einer Person und der Gasaustauschkammer auf einfache Weise zu realisieren. Insbesondere ist die Gasaustauschkammer bei deaktivierten Pumpen betreibbar, ohne den Blutkreislauf der Person zu beeinflussen.

Die Verwendung von Pumpen, die im deaktivierten Zustand einen Fluss verhindern, bietet den Vorteil zwischen dem Blutkreislauf und der Gasaustauschkammer eine effektive Trennung zu realisieren, ohne weitere Ventile zu benötigen. Dies ermöglicht eine platzsparende, einfache und wartungsarme Realisierung eines Systems zur extrakorporalen Elimination von Kohlenmonoxid.

In einer weiteren Ausführung der Erfindung ist es vorgesehen, dass zwischen dem zweiten Schlauchabschnitt und der Gasaustauschkammer ein Blasenfilter angeordnet ist.

Die Verwendung eines Blasenfilters führt dazu, dass im aufbereiteten Blut befindliche Gasblasen herausgefiltert werden. Insbesondere führt ein Blasenfilter dazu, dass das Blut aus der Gasaustauschkammer, welches nach der Elimination von Kohlenmonoxid und der Sauerstoffanreicherung Gasblasen aufweisen kann, vor der Zurückführung in den Blutkreislauf der Person von diesen Gasblasen befreit wird.

Dies hat den Vorteil, dass blasenfreies Blut aus der Gasaustauschkammer in den Blutkreislauf der Person zurückgeführt wird und dadurch negative Auswirkungen durch zu große Gasblasen im Blutkreislauf verhindert werden.

Eine vorteilhafte Ausführung der Erfindung sieht vor, dass der Blasenfilter zwischen dem zweiten Schlauchabschnitt und der Gasaustauschkammer Poren mit einem Durchmesser im Bereich von 5 bis 80 µm aufweist. Durch Poren dieser Größe werden Gasblasen, die einen größeren Durchmesser aufweisen, am Durchfluss behindert. Beispielsweise kann ein entsprechender Blasenfilter durch eine Membran oder einen Schaum oder ein Textil gebildet sein. Ein Kunststoffgewebe ist besonders bevorzugt als Blasenfilter einzusetzen. Zudem ist es vorgesehen den Blasenfilter über eine Verschraubung und/oder eine Arretiervorrichtung an die Gasaustauschkammer anzubringen. Eine weitere Ausführung kann es vorsehen, dass der Blasenfilter in die Gasaustauschkammer integriert ist.

Eine derartige Ausführung des Blasenfilter führt vorteilhafterweise dazu, dass das Blut, das in den Blutkreislauf der Person zurückgeführt wird nur Sauerstoffblasen aufweist, die zu keiner negativen Beeinträchtigung im Blutkreislauf führen. Durch die Befestigung mittels einer Verschraubung und/oder Arretiervorrichtung ist es vorteilhafterweise möglich den Blasenfilterbei Bedarf einfach auszutauschen.

Gemäß der Erfindung ist vorgesehen, dass die Gasaustauschkammer eine Sauerstoffzuführung aufweist, die über eine Sauerstoffzuleitung mit einer Sauerstoffquelle verbunden ist. Die Sauerstoffquelle beinhaltet bevorzugt ein sauerstoffhaltiges Gasgemisch mit einer hohen Sauerstoffkonzentration, insbesondere mit einer Sauerstoffkonzentration von über 95 %.

Auf diese Weise wird der Gasaustauschkammer, in der sich das mit Kohlenmonoxid beladene Hämoglobin befindet, Sauerstoff hinzugefügt. Die Vermischung des mit Kohlenmonoxid beladenen Hämoglobins mit Sauerstoff führt dazu, dass das Kohlenmonoxid durch Sauerstoff ersetzt wird und damit eine Elimination von Kohlenmonoxid aus dem Blut stattfindet. Ein Kontakt zwischen Sauerstoff und mit Kohlenmonoxid belegtem Hämoglobin hat den Vorteil, dass ein direkter Austausch stattfindet und auf weitere Komponenten, wie zum Beispiel Membranen, verzichtet werden kann.

Durch die Zuführung von Sauerstoff und den direkten Kontakt zwischen Sauerstoff und Blut ist die erfindungsgemäße Gasaustauschkammer einfach zu realisieren und im Vergleich zu anderen Technologien wartungsarm.

Die Erfindung sieht vor, dass die Gasaustauschkammer mit einem Druck über dem umgebenden Atmosphärendruck beaufschlagbar ist, z.B. 0,5 bis 10 bar darüber.

Die Beaufschlagung der Gasaustauschkammer mit einem Druck führt dazu, dass der Kontakt zwischen dem mit Kohlenmonoxid beladenem Hämoglobin und dem Sauerstoff bei einem Druck stattfindet der den Austausch Kohlenmonoxids durch Sauerstoff positiv beeinflusst.

Vorteilhafterweise führt die Beaufschlagung der Gasaustauschkammer mit einem Druck zu einer effektiveren und schnelleren Elimination des Kohlenmonoxids im Blut und damit zu einer schnelleren Behandlung des Patienten.

In einer besonders praktischen Ausführung der Erfindung ist die Sauerstoffquelle eine Sauerstoffflasche. In dieser besonders praktischen Ausführung ist die Sauerstofflasche mit reinem Sauerstoff oder einem Gasgemisch mit sehr hohem Sauerstoffanteil gefüllt. Die Verwendung einer Sauerstoffflasche bietet die Möglichkeit der Gasaustauschkammer über die Sauerstoffzuleitung und die Sauerstoffzuführung Sauerstoff zuzuführen und damit den Austausch mit dem Kohlenmonoxid zu realisieren. Eine Gasflasche hat dabei den Vorteil, dass diese Druck aufbauen kann und zudem typischerweise in Rettungsfahrzeugen oder Behandlungsräumen vorliegt. Außerdem werden Sauerstoffflaschen in einer sehr hohen Stückzahl hergestellt, so dass sich auch eine günstige Lösung für ein System zur extrakorporalen Elimination von Kohlenmonoxid realisieren lässt. Weiterhin sind Sauerstofflaschen auch in kleiner Bauform verfügbar, was das System mobil transportierbar macht.

Weiterhin ist die Erfindung dadurch gekennzeichnet, dass die Sauerstoffzuführung ein Ausströmelement aufweist, das zur Bildung von Gasblasen eingerichtet ist.

Durch die Verwendung eines Ausströmelementes, das zur Bildung von Gasblasen eingerichtet ist, werden im Blut, das sich in der Gasaustauschkammer befindet, viele Gasblasen gebildet und somit die effektive Wechselwirkungsfläche zwischen Sauerstoff und Blut erhöht.

Die Vergrößerung der effektiven Wechselwirkungsfläche zwischen Blut und Sauerstoff führt dazu, dass ein Austausch des Kohlenmonoxids durch Sauerstoff schneller stattfindet und damit eine effiziente Elimination von Kohlenmonoxid im Blut realisierbar ist.

In einer besonders praktikablen Ausführung der Erfindung ist es vorgesehen, dass das Ausströmelement eine perforierte Leitung, eine perforierte Membran oder ein poröser Schaum ist.

Durch die Verwendung einer perforierten Leitung, einer perforierten Membran oder eines porösen Schaumes ist es möglich auf effektive Weise Gasblasen in der Gasaustauschkammer herzustellen und auf diese Weise einen möglichst effektiven Austausch von Kohlenmonoxid durch Sauerstoff zu gewährleisten. Die Verwendung einer perforierten Leitung, einer perforierten Membran oder eines porösen Schaumes als Ausströmelement hat den Vorteil, dass auf einfache Weise mit üblichen Bauelementen die gewünschten Gasblasen im Blut der Gasaustauschkammer herzustellen sind.

Eine weitere Ausführung der Erfindung sieht es vor, dass die Gasaustauschkammer eine Kohlenmonoxidableitung aufweist.

Durch die Kohlenmonoxidableitung wird das Kohlenmonoxid, das im Blut durch Sauerstoff ersetzt worden ist und dadurch im gasförmigen Zustand in der Gasaustauschkammer befindlich ist, aus dieser abgeleitet. Über die Kohlenmonoxidableitung wird zudem der Sauerstoff abgeführt, der nicht durch das Hämoglobin des Blutes in der Gasaustauschkammer aufgenommen worden ist. Auf diese Weise werden die gasförmigen Abfallprodukte der Elimination von Kohlenmonoxid aus der Gaskammer abgeführt.

Vorteilhafterweise wird dadurch die Konzentration des Kohlenmonoxids in der Gasaustauschkammer im Vergleich zur Sauerstoffkonzentration gering gehalten und dadurch ein effektiver Austausch zwischen Kohlenmonoxid und Sauerstoff erreicht.

In einer weiteren bevorzugten Ausführungsvariante der Erfindung ist es vorgesehen, dass zwischen Gasaustauschkammer und Kohlenmonoxidableitung ein Schaumfilter angeordnet ist. Es kann vorteilhafterweise auch vorgesehen sein, dass der Schaumfilter in die Gasaustauschkammer integriert ist. Eine weitere Ausführung kann zudem vorsehen, dass der Schaumfilter auswechselbar an der Gasaustauschkammer angeordnet ist.

Der Schaumfilter dient dazu, einen Austritt von schaumförmigem Blut, das sich durch Zuführung der Sauerstoffblasen in der Gasaustauschkammer bildet, zu unterbinden.

Vorteilhafterweise wird somit ein Austritt von Blut und dadurch ein Blutverlust durch das System zur extrakorporalen Elimination von Kohlenmonoxid verhindert.

Eine weitere Ausführung der Erfindung sieht es vor, dass die Sauerstoffzuleitung ein erstes Ventil und die Kohlenmonoxidableitung ein zweites Ventil aufweist, die insbesondere jeweils ansteuerbar sind.

Die Verwendung dieser beiden Ventile macht es möglich je nach Bedarf Sauerstoff in die Gasaustauschkammer zuzuführen und/oder Kohlenmonoxid und Sauerstoff aus der Gasaustauschkammer abzuführen. Dadurch ist der Druck innerhalb der Gasaustauschkammer und der Sauerstoffdurchfluss in Form von Gasblasen durch das Blut steuerbar, so dass eine effektive Elimination von Kohlenmonoxid einstellbar ist.

Vorteilhafterweise ermöglichen das erste und das zweite Ventile eine optimierte Einstellung des Drucks und des Sauerstoffdurchflusses und der Sauerstoffkonzentration in der Gasaustauschkammer sowie eine portionsweise Behandlung des Blutes innerhalb dieser für eine effektive und schnelle Elimination von Kohlenmonoxid vom Hämoglobin und einer Sauerstoffanreicherung am Hämoglobin.

Gemäß einer weiteren Ausführungsvariante der Erfindung weist der erste Schlauchabschnitt oder der zweite Schlauchabschnitt zumindest ein drittes Ventil auf. Vorzugsweise ist das dritte Ventil zwischen dem Blasenfilter und der Pumpe angeordnet.

Mit einem dritten Ventil, das sich zwischen dem Blutkreislauf der Person und der Gasaustauschkammer befindet, lässt sich ein Fluss zwischen dem Blutkreislauf und der Gasaustauschkammer verhindern. Insbesondere ermöglicht ein drittes Ventil eine druckdichte Verschließung der Gasaustauschkammer, sodass diese mit einem für die Elimination von Kohlenmonoxid optimalen Druck beaufschlagbar ist.

Die Verwendung eines dritten Ventils bietet den Vorteil, dass der Blutkreislauf der Person auf einfache Weise von der Gasaustauschkammer zu trennen ist. Insbesondere ermöglicht ein drittes Ventil den Aufbau des erfindungsgemäßen Systems mit solchen Pumpen, die im deaktivierten Zustand einen Fluss nicht verhindern. Die Anordnung des dritten Ventils zwischen dem Blasenfilter und der Pumpe bietet den Vorteil, dass eine Druckvariation durch einen Betrieb des Ventils im ersten Schlauchabschnitt und damit auf den Blutkreislauf der Person reduziert oder verhindert wird.

In einer weiteren Ausführungsvariante der Erfindung ist es vorgesehen, dass die Gasaustauschkammer eine erste Sensoreinheit aufweist, mit der eine Kohlenmonoxidkonzentration bestimmbar ist. Z.B. kann diejenige im Blut und/oder die im Gas gemessen werden.

Die Verwendung eines Kohlenmonoxidsensors bietet die Möglichkeit die Ventile der Sauerstoffzuleitung und/oder der Kohlenmonoxidableitung gezielt, insbesondere also in Abhängigkeit der gemessenen Konzentration von Kohlenmonoxid zu steuern und so eine gewünschte Kohlenmonoxidkonzentration in der Gasaustauschkammer zu realisieren. Insbesondere lässt sich in Abhängigkeit von der Kohlenmonoxidkonzentration feststellen wann die Elimination des Kohlenmonoxids aus dem Blut soweit fortgeschritten ist, dass das mit Sauerstoff angereicherte Blut wieder in den Blutkreislauf der Person zurückführbar ist und somit die vorteilhafte portionsweise Behandlung des Blutes nach Bedarf möglich ist.

Ein Kohlenmonoxidsensor hat somit den Vorteil, dass auf Grundlage der bestimmten Kohlenmonoxidkonzentration der Prozess zur Elimination von Kohlenmonoxid einstellbar und optimierbar ist.

In einer weiteren bevorzugten Ausführungsvariante der Erfindung ist es vorgesehen, dass die Gasaustauschkammer eine zweite Sensoreinheit aufweist, mit der ein Druck innerhalb der Gasaustauschkammer messbar ist.

Mittels des durch den Drucksensors bestimmten Druckes innerhalb der Gasaustauschkammer lässt sich die Zuführung von Sauerstoff über die Sauerstoffzuleitung und die Abführung von Kohlenmonoxid über die Kohlenmonoxidableitung derart einstellen, dass ein gewünschter Druck erreicht werden kann und für eine Zeit gehalten werden kann oder ein Druckverlauf abgefahren werden kann, insbesondere so eine optimale Elimination von Kohlenmonoxid stattfindet.

In einer weiteren Ausführungsvariante der Erfindung weist das System ein Durchflussmesser auf, der die Blutmenge die in die Gasaustauschkammer hineingeführt und/oder herausgeführt wird bestimmt. Zudem kann es in einer Ausführung vorgesehen sein an die Sauerstoffzuleitung einen Sauerstoffdurchflusssensor und/oder Sauerstoffkonzentrationssensor und/oder an die Kohlenmonoxidableitung einen Kohlenmonoxiddurchflusssensor und/oder Kohlenmonoxidkonzentrationssensor anzuordnen. Vorteilhafterweise ist in der Gasaustauschkammer ein Füllstandsensor integriert, der den Blutfüllstand innerhalb der Gasaustauschkammer bestimmt.

Die Verwendung dieser zusätzlichen Sensoren erlaubt es das erfindungsgemäße System optimal zu steuern und eine möglichst effiziente Elimination von

Kohlenstoffmonoxid zu erzielen. Beispielsweise kann der Sauerstoffzufluss und der Kohlenmonoxidabfluss auf einen identischen Wert eingestellt werden, so dass bei konstantem Druck, der insbesondere über dem Atmosphärendruck liegt, eine kontinuierliche Erzeugung von Gasblasen erzielt wird.

In einer besonders bevorzugten Ausführungsvariante der Erfindung ist das System dazu eingerichtet das Blut portionsweise zu behandeln, wobei in einem ersten Betriebsmodus Blut aus dem Blutkreislauf der Person in die Gasaustauschkammer befördert wird. Beispielsweise wird dazu im ersten Betriebsmodus das erste Ventil ganz oder teilweise geschlossen, das zweite Ventil ganz oder teilweise geöffnet und die Pumpe so angesteuert, dass das Blut in die Gasaustauschkammer befördert wird. Dadurch, dass das zweite Ventil geöffnet wird kann das Blut, das durch die Pumpe in Richtung Gasaustauschkammer geführt wird in diese hineinfließen ohne dass sich in der Kammer ein Gegendruck aufbaut. Es kann insbesondere von Vorteil sein, wenn beim Hineinbefördern des Blutes in die Gasaustauschkammer kein oder wenig Sauerstoff eingeführt wird, um eine unnötige Vergeudung von Sauerstoff zu vermeiden.

Eine weitere Ausführung der Erfindung sieht es vor, dass das System dazu eingerichtet ist Blut portionsweise zu behandeln, wobei in einem zweiten Betriebsmodus Blut in der Gasaustauschkammer unter erhöhtem Druck mit Sauerstoff in Kontakt gebracht wird. Beispielsweise wird im zweiten Betriebsmodus die Pumpe deaktiviert und der Öffnungsgrad des ersten Ventils sowie des zweiten Ventils variiert. , wobei in einer besonders praktischen Ausführung die Steuerung des Öffnungsgrades des ersten Ventils und/oder des zweiten Ventils nichtplötzlich sondern zeitlich kontrolliert stattfinden. Zum Druckaufbau innerhalb der Gasaustauschkammer wird beispielsweise das erste Ventil ganz oder teilweise geöffnet und das zweite Ventil ganz oder Teilweise geschlossen. Für eine kontinuierliche Zuführung von Sauerstoff bei Konstantem Druck wird der Öffnungsgrade des ersten Ventils und des zweiten Ventils so eingestellt, dass der Sauerstoffzufluss und der Kohlenmonoxidabfluss einen identischen Wert einnehmen. Zum Druckabbau innerhalb der Gasaustauschkammer wird beispielsweise das erste Ventil ganz oder teilweise geschlossen und das zweite Ventil ganz oder teilweise geöffnet.

Dadurch dass die Pumpe deaktiviert ist und das zweite Ventil ganz oder teilweise geschlossen ist, kann aus der Gasaustauschkammer keine Flüssigkeit entweichen. Durch das ganz oder teilweise Öffnen des ersten Ventils wird Sauerstoff in die Gasaustauschkammer hineingeführt und ein erhöhter Druck in der Gasaustauschkammer gebildet. Zudem ist durch eine gezielte Einstellung einer Sauerstoffzufuhr durch das erste Ventil und eine gezielte Einstellung einer Kohlenstoffmonoxidabfuhr durch das zweite Ventil ein kontinuierlicher Sauerstoffdurchfluss bei einem bestimmten Druck realisierbar, so dass eine zeitlich konstante Sauerstoffblasenbildung zu realisieren ist. Das Ablassen des Druckes innerhalb der Gasaustauschkammer über das zweite Ventil ermöglicht es in der Gasaustauschkammer den Druck wieder an den Atmosphärendruck anzupassen, so dass der Druck innerhalb Gasaustauschkammer an den Druck des Blutkreislaufs angeglichen ist. Der zweite Betriebsmodus bietet auf diese Weise den Vorteil, dass eine effektive Elimination von Kohlenmonoxid in der Gasaustauschkammer bei erhöhtem Druck mit einer hohen Sauerstoff-Blut-Kontaktfläche effektiv durchführbar ist.

Das zweite Ventil ist in einer bevorzugten Ausführung ein Überdruckventil, das automatisch Druck ablässt, wenn der gewünschte Druck durch die Sauerstoffzuführung überschritten wird, so dass der Druck unter Zuführung von Sauerstoff konstant bleibt.

In einer weiteren Ausführung der Erfindung ist es vorgesehen, dass das System dazu eingerichtet ist Blut portionsweise zu behandeln, wobei in einem dritten Betriebsmodus Blut aus der Gasaustauschkammer in den Blutkreislauf der Person zurück befördert wird. Beispielsweise wird in diesem Betriebsmodus das erste Ventil ganz oder teilweise geschlossen, das zweite Ventil ganz oder teilweise geöffnet und die Pumpe so angesteuert, dass das Blut aus der Gasaustauschkammer in den Blutkreislauf der Person befördert wird. In einer besonders praktischen Ausführung können das Schließen des ersten Ventils und/oder das Öffnen des zweiten Ventils nicht plötzlich, sondern zeitlich kontrolliert stattfinden.

Bei der Zurückführung des Blutes in den Blutkreislauf ist das erste Ventil, also die Sauerstoffzuführung, geschlossen um unnötigen Verbrauch von Sauerstoff zu vermeiden. Das zweite Ventil in der Kohlenmonoxidableitung ist im dritten Betriebsmodus beispielsweise geöffnet, um eine einfachere Zurückführung des Blutes in den Blutkreislauf zu realisieren.

Die portionsweise Behandlung von Blut durch aufeinander folgende Durchführung des ersten, des zweiten und des dritten Betriebsmodus ermöglicht vorteilhafterweise eine effektive Elimination von Kohlenmonoxid, wobei nur ein Zugang zum Blutkreislauf der Person anzulegen ist. Dadurch ist eine minimal invasive und schnelle Inbetriebnahme des erfindungsgemäßen Systems an der Person möglich. Eine wiederholte Durchführung der portionsweisen Behandlung von Blut durch die aufeinander folgende Durchführung des ersten, zweiten und des dritten Betriebsmodus ermöglicht vorteilhafterweise die schrittweise effektive Elimination von Kohlenmonoxid aus dem Blutkreislauf der Person.

Eine besonders praktikable Ausführungsvariante der Erfindung sieht es vor, dass das System eine Steuereinheit aufweist, die dazu eingerichtet ist die Pumpe und/oder das erste Ventil und/oder das zweite Ventil zu steuern und/oder die erste Sensoreinheit und/oder die zweite Sensoreinheit auszulesen.

Die Verwendung einer Steuereinheit, die die Komponenten des Systems je nach Bedarf steuert und/oder ausliest, ermöglicht eine effiziente Steuerung des erfindungsgemäßen Systems. Die Steuereinheit ist insbesondere dazu ausgelegt aufeinanderfolgend den ersten Betriebsmodus, den zweiten Betriebsmodus und den dritten Betriebsmodus durchzuführen. Zudem lassen sich die Betriebsmodi durch Auswertung des Drucks und der Kohlenmonoxidkonzentration über die erste und die zweite Sensoreinheit dahingehend optimieren, dass die Elimination von Kohlenmonoxid möglichst effektiv durchzuführen ist.

Eine besonders anwenderfreundliche Ausführung der Erfindung sieht es vor, dass das System in einem tragbaren Koffer mobil transportierbar ist sowohl im Nichtbetrieb als auch im Betrieb an einer Person.

Dadurch, dass das System in einem Koffer untergebracht werden kann und transportierbar ist, wird sichergestellt, dass das erfindungsgemäße System zur Elimination von Kohlenmonoxid in Krankenwagen oder Rettungswagen oder Notarztwagen mobil zu transportieren ist. Damit wird vorteilhafterweise sichergestellt, dass eine Elimination von Kohlenmonoxid auch ortsnah außerhalb eines Krankenhauses an einem Unfallort und direkt durchführbar ist, so das eine schnelle Behandlung der Patienten gewährleist ist und damit Folgeschäden durch die Kohlenmonoxidvergiftung reduziert werden.

Im Folgenden werden Ausführungen der Erfindung anhand von Figuren vorgestellt und näher erläutert. Es zeigen:
- Fig. 1: den schematischen Aufbau des erfindungsgemäßen Systems mit einer bidirektionalen Pumpe,
- Fig. 2: in einer schematischen Darstellung das erfindungsgemäße System mit zwei unidirektionalen Pumpen.

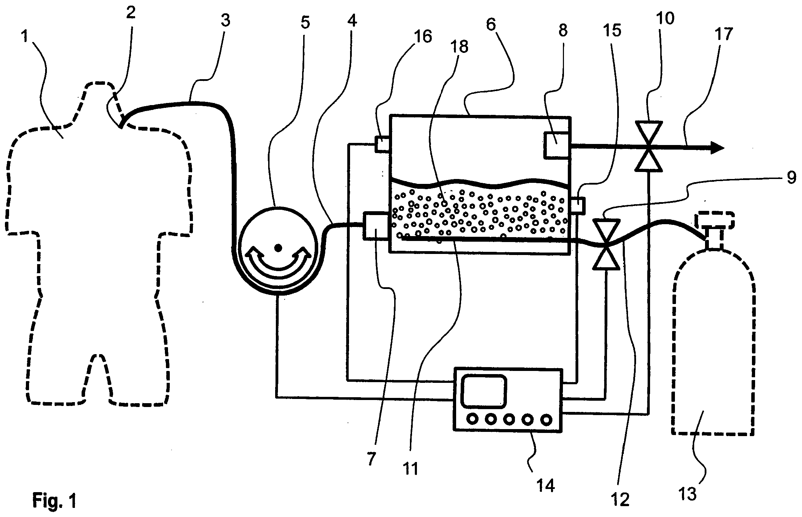

In Fig. 1 ist das erfindungsgemäße System zur extrakorporalen Elimination von Kohlenmonoxid in einer schematischen Abbildung dargestellt, wobei die Person 1 über eine Kanüle 2 und über einen Schlauch, der einen ersten Schlauchabschnitt 3 und einen zweiten Schlauchabschnitt 4 aufweist, mit der Gasaustauschkammer 6 verbindbar ist. Als Pumpe 5 wird in diesem Ausführungsbeispiel eine bidirektionale Pumpe 5 eingesetzt, die entweder Blut aus dem Blutkreislauf der Person 1 in die Gasaustauschkammer 6 befördert oder Blut aus der Gasaustauschkammer 6 in den Blutkreislauf der Person 1 zurück befördert oder einen Fluss unterbindet. Dadurch ist es möglich mittels einer einzigen, durch die Kanüle 2 gebildeten Anschlussstelle an den Blutkreislauf der Person 1 ein System zur extrakorporalen Elimination von Kohlenmonoxid zu realisieren.

Die Pumpe 5 ist in einer bevorzugten Ausführungsform eine Schlauchpumpe, die bidirektional betreibbar ist. Eine Schlauchpumpe bietet auf einfache Weise die Möglichkeit bidirektional betrieben zu werden und einen Fluss zwischen dem ersten Schlauchabschnitt 3 und dem zweiten Schlauchabschnitt 4 im deaktivierten Zustand zu verhindern. Außerdem kommt nur der erste Schlauchabschnitt 3 und der zweite Schlauchabschnitt 4 in Kontakt mit dem Blut, sodass eine Reinigung der Pumpe entfällt, was insbesondere aus hygienischen Gründen Vorteile aufweist.

In der in Fig. 1 dargestellten Ausführungsvariante ist zwischen dem zweiten Schlauchabschnitt 4 und der Gasaustauschkammer 6 ein Blasenfilter 7 angeordnet, der verhindert dass Gasblasen 18, die in der Gasaustauschkammer 6 gebildet werden in den Blutkreislauf der Person 1 zurückgeführt werden. Vorteilhafterweise weist der Blasenfilter 7 Poren mit einem Durchmesser im Bereich von 5 µm bis 80 µm auf, so dass die Gasblasen 18, die ggf. in dem Blutkreislauf der Person 1 zurückgeführt werden, dort keine negativen Auswirkungen haben. Der Blasenfilter 7 ist dabei vorzugsweise eine Membran oder ein Schaum oder ein Textil. Ein Kunststoffgewebe ist als Blasenfilter besonders bevorzugt.

Die in Fig. 1 dargestellt Ausführungsform der Erfindung weist eine Sauerstoffzuführung 11 auf, die über eine Sauerstoffzuleitung 12 mit einer Sauerstoffquelle 13 verbunden ist. Durch die Sauerstoffzuführung wird Sauerstoff in die Gasaustauschkammer 6 hineingeführt und damit eine hohe Sauerstoffkonzentration innerhalb der Gasaustauschkammer 6 erzeugt. Dies führt dazu, dass das mit Kohlenmonoxid beladene Hämoglobin mit Sauerstoff in Kontakt kommt und das Kohlenmonoxid durch Sauerstoff ausgetauscht wird. Auf diese Weise findet in der Gasaustauschkammer 6 eine Elimination von Kohlenmonoxid im Blut statt. Vorteilhafterweise wird die Gasaustauschkammer 6 mit einem Druck im Bereich von 0,5 bar bis 10 bar über dem umgebenden Atmosphärendruck beaufschlagt, so dass die Elimination bei erhöhtem Druck stattfindet und damit effektiver abläuft. Als Sauerstoffquelle 13 wird bevorzugt eine Sauerstoffflasche eingesetzt, die zum einen den notwendigen Druck aufbringt und zum anderen sowohl in Krankenwagen als auch in Behandlungsräumen üblicherweise zu Verfügung steht.

In einer nicht dargestellten Ausführungsform der Erfindung weist die Sauerstoffzuführung 11 ein Ausströmelement auf, das zur Bildung von Gasblasen 18 eingerichtet ist. Das Ausströmelement kann durch eine perforierte Leitung, eine perforierte Membran oder durch einen porösen Schaum gebildet sein.

Wie in Fig. 1 dargestellt, weist die Gasaustauschkammer 6 in einer Ausführungsvariante eine Kohlenmonoxidableitung 17 auf, über die Kohlenmonoxid und/oder Sauerstoff aus der Gasaustauschkammer 6 entweichen kann. Des Weiteren ist zwischen der Gasaustauschkammer 6 und Kohlenmonoxidableitung 17 ein Schaumfilter 8 angeordnet, der dafür sorgt dass tatsächlich nur Gase, also Sauerstoff und/oder Kohlenmonoxid aus der Gasaustauschkammer 6 entweichen. Das in der Gasaustauschkammer 6 befindliche mit Gasblasen 18 versehene Blut wird somit nicht aus der Gasaustauschkammer 6 geführt.

Die Sauerstoffzuleitung 12 weist in der dargestellten Ausführungsvariante ein erstes Ventil 9 auf. Durch das erste Ventil 9 ist die zugeführte Sauerstoffmenge in der Gasaustauschkammer 6 steuerbar. Des Weiteren weist die Kohlenmonoxidableitung 17 ein zweites Ventil 10 auf, mit dem der Druck innerhalb der Gasaustauschkammer 6 und/oder die Abführung von Kohlenmonoxid und/oder Sauerstoff steuerbar sind. Dadurch wird sichergestellt, dass die prozessbedingten Abfallstoffe aus der Gasaustauschkammer 6 abgeführt werden und der Druck innerhalb der Gasaustauschkammer 6 regulierbar ist.

In einer nicht dargestellten Ausführungsvariante weist der erste Schlauchabschnitt 3 oder der zweite Schlauchabschnitt 4 zumindest ein drittes Ventil auf. Mittels eines dritten Ventils ist somit ein Fluss zwischen dem Blutkreislauf der Person 1 und der Gasaustauschkammer 6 verhinderbar, so dass für das erfindungsgemäße System auch Pumpen eingesetzt werden können, die im nichtpumpenden Zustand einen Fluss zulassen. Zudem ermöglicht die Verwendung eines dritten Ventils die Beaufschlagung der Gasaustauschkammer 6 mit einem höheren Druck. Das dritte Ventil wird bevorzugt zwischen der Pumpe 5 und dem Blasenfilter 7 angeordnet.

Wie in Fig. 1 dargestellt, ist an der Gasaustauschkammer 6 eine erste Sensoreinheit 15 angeordnet, mit der eine Kohlenmonoxidkonzentration bestimmbar ist, wobei durch die Messung der Kohlenmonoxidkonzentration eine optimale Steuerung des Systems realisiert werden kann. Insbesondere wird durch die erste Sensoreinheit 15 sichergestellt, dass nur Blut in den Blutkreislauf der Person 1 zurückgeführt wird, das eine ausreichende Sauerstoffkonzentration und/oder eine ausreichend geringe Kohlenmonoxidkonzentration aufweist. In der dargestellten Ausführungsform weist die Gasaustauschkammer 6 eine zweite Sensoreinheit 16 auf, mit der ein Druck innerhalb der Gasaustauschkammer 6 messbar ist. Dieser Drucksensor ermöglicht es den Druck innerhalb der Kammer optimal einzustellen. Insbesondere lässt sich der Öffnungsgrad des ersten Ventils 9 und/oder des zweiten Ventiles 10 variabel einstellen, um einen optimalen Druck und/oder einen optimalen Druckverlauf in der Gasaustauschkammer 6 zu gewährleisten.

In einer nicht dargestellten weiteren Ausführung der Erfindung weist das System zur Elimination von Kohlenmonoxid ein Durchflussmesser auf, der die Blutmenge die in die Gasaustauschkammer hineingeführt und/oder herausgeführt wird bestimmt. Zudem kann es in einer Ausführung vorgesehen sein an die Sauerstoffzuleitung einen Sauerstoffdurchflusssensor und/oder Sauerstoffkonzentrationssensor und/oder an die Kohlenmonoxidableitung einen Kohlenmonoxiddurchflusssensor und/oder Kohlenmonoxidkonzentrationssensor anzuordnen. Vorteilhafterweise ist in der Gasaustauschkammer zudem ein Füllstandsensor integriert, der den Blutfüllstand innerhalb der Gasaustauschkammer bestimmt. Die Verwendung dieser zusätzlichen Sensoren ermöglicht es das erfindungsgemäße System optimal zu steuern und eine möglich effiziente Elimination von Kohlenstoffmonoxid zu erzielen. Sauerstoffzufluss und der Kohlenmonoxidabfluss können beispielsweise auf einen identischen Wert eingestellt werden, so dass bei konstantem Druck, der insbesondere über dem Atmosphärendruck liegt, eine kontinuierliche Erzeugung von Blasen erzielt wird.

Wie in Fig. 1 dargestellt, weist eine Ausführungsvariante der Erfindung eine Steuereinheit 14 auf, die dazu eingerichtet ist die Pumpe 5 und/oder das erste Ventil 9 und/oder das zweite Ventil 10 zu steuern und/oder die erste Sensoreinheit 15 und/oder die zweite Sensoreinheit 16 auszulesen. Die Steuereinheit 14 ermöglicht somit eine effektive Steuerung des Systems, wobei bevorzugter Weise drei Betriebsmodi zu unterscheiden sind.

Bei dem ersten Betriebsmodus wird das Blut aus dem Blutkreislauf der Person 1 in die Gasaustauschkammer 6 befördert. Dies kann beispielsweise dadurch erreicht werden, dass das erste Ventil 9 ganz oder teilweise geschlossen wird, das zweite Ventil 10 ganz oder teilweise geöffnet ist und die Pumpe 5 so angesteuert wird, dass diese das Blut in die Gasaustauschkammer 6 befördert.

Im zweiten Betriebsmodus wird das Blut in der Gasaustauschkammer 6 unter erhöhtem Druck mit Sauerstoff in Kontakt gebracht. Dazu wird beispielsweise die Pumpe 5 deaktiviert, sodass ein Fluss zwischen erstem Schlauchabschnitt 3 und dem zweiten Schlauchabschnitt 4 verhindert ist, zudem wird der Öffnungsgrad des ersten Ventils 9 und des zweiten Ventil 10 variiert, wobei das Öffnen und Schließen plötzlich oder zeitlich kontrolliert passieren kann. Im zweiten Betriebsmodus wird somit durch die Sauerstoffzuführung 11 über die Sauerstoffzuleitung 12 aus der Sauerstoffquelle 13 Sauerstoff in die Gasaustauschkammer 6 eingeführt, zudem wird die Gasmischung aus zugeführtem Sauerstoff und aus dem Blut eliminierten Kohlenmonoxid über die Kohlenmonoxidableitung 17 abgeführt. Als Folge dessen herrscht in der Gasaustauschkammer 6 eine hohe Sauerstoffkonzentration und gleichzeitig wird Kohlenmonoxid über die Kohlenmonoxidableitung 17 aus der Gasaustauschkammer 6 abgeführt, sodass eine effektive Elimination von Kohlenmonoxid realisiert ist.

Im dritten Betriebsmodus wird das Blut aus der Gasaustauschkammer 6 in den Blutkreislauf der Person 1 zurück gefördert. Beispielsweise wird dafür das erste Ventil 9 ganz oder teilweise geschlossen, das zweite Ventil 10 ganz oder teilweise geöffnet, wobei das Schließen und Öffnen plötzlich oder zeitlich kontrolliert passieren kann, und die Pumpe 5 so angesteuert, dass das Blut aus der Gasaustauschkammer 6 in den Blutkreislauf der Person 1 befördert wird. Somit wird sichergestellt, dass mit Sauerstoff angereichertes Blut zurück in den Blutkreislauf der Person 1 geführt wird.

Eine wiederholte Ausführung des ersten Betriebsmodus, des zweiten Betriebsmodus und des dritten Betriebsmodus führt zu einer portionsweisen Behandlung des Blutes und ermöglicht auf diese Weise eine minimal invasive Elimination von Kohlenmonoxid mit einem Zugang, bzw. einer Kanüle 2, an den Blutkreislauf der Person 1.

In einer nicht explizit dargestellten Ausführungsform der Erfindung ist das System so ausgelegt, dass es in einem tragbaren Koffer Platz findet und mobil transportierbar ist. Auf diese Weise ist es möglich das erfindungsgemäße System in Krankenwagen oder Rettungswagen oder Krankenhäusern und Praxen vor Ort einzusetzen.

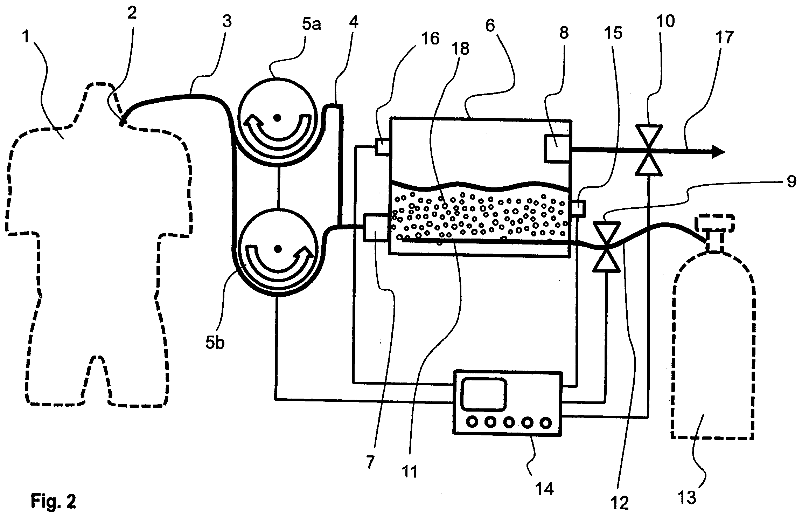

In Fig. 2 ist eine schematische Darstellung der Erfindung mit zwei unidirektional arbeitenden Pumpen 5a und 5b dargestellt. Eine derartige Ausführung der Erfindung bietet die Möglichkeit auch unidirektionale Pumpen 5a und 5b zur Realisierung des erfindungsgemäßen Systems einzusetzen. Die Pfeile geben dabei die Flussrichtung in aktivierten Zustand der Pumpen 5a und 5b an. Dabei können unidirektionale Pumpen 5a und 5b den Vorteil haben, dass sie günstiger zu beschaffen sind und eine längere Lebensdauer aufweisen. Bei der in Fig. 2 dargestellten Ausführungsvariante ist die Steuereinheit 14 dazu eingerichtet, die Pumpen 5a und 5b und/oder das erste Ventil 9 und/oder das zweite Ventil 10 zu steuern und/oder die erste Sensoreinheit 15 und/oder zweite Sensoreinheit 16 auszulesen. Im ersten Betriebsmodus wird beispielsweise die erste Pumpe 5a deaktiviert, die zweite Pumpe 5b aktiviert, das erste Ventil 9 ganz oder teilweise geschlossen und das zweite Ventil 10 ganz oder teilweise geöffnet. Auf diese Weise wird sichergestellt, dass das Blut aus dem Blutkreislauf der Person 1 in die Gasaustauschkammer 6 befördert wird. Im zweiten Betriebsmodus werden beide Pumpen 5a und 5b deaktiviert und der Öffnungsgrad des ersten Ventils 9 und des zweiten Ventils 10 variiert. Dadurch wird Sauerstoff in die Gasaustauschkammer 6 hinein befördert, sodass bei erhöhtem Druck eine Elimination von Kohlenmonoxid stattfindet. Im dritten Betriebsmodus wird die erste Pumpe 5a aktiviert, die zweite Pumpe 5b deaktiviert, das erste Ventil 9 ganz oder teilweise geschlossen und das zweite Ventil 10 ganz oder teilweise geöffnet. Auf diese Weise wird im dritten Betriebsmodus eine Zurückbeförderung des Blutes in den Blutkreislauf der Person 1 realisiert. Bei den in Fig. 2 dargestellten Pumpen 5a und 5a handelt es sich bevorzugt um Pumpen, die im deaktivierten Zustand einen Fluss zwischen dem ersten Schlauchabschnitt 3 und dem zweiten Schlauchabschnitt 4 verhindern.

Das erfindungsgemäße System zur extrakorporalen Elimination von Kohlenmonoxid ermöglicht es mit einem kompakten Aufbau und auf einfache Weise Blut effektiv portionsweise zu behandeln und damit eine schnelle und minimal invasive Therapie von Kohlenmonoxidvergiftungen sofort am Unfallort und/oder beim Transport des Patienten zu realisieren.

### Bezugszeichen

- 1: Person
- 2: Kanüle
- 3: erster Schlauchabschnitt
- 4: zweiter Schlauchabschnitt
- 5: Pumpe
- 5a: erste unidirektionale Pumpe
- 5b: zweite unidirektionale Pumpe
- 6: Gasaustauschkammer
- 7: Blasenfilter
- 8: Schaumfilter
- 9: erstes Ventil
- 10: zweites Ventil
- 11: Sauerstoffzuführung
- 12: Sauerstoffzuleitung
- 13: Sauerstoffquelle
- 14: Steuereinheit
- 15: erste Sensoreinheit
- 16: zweite Sensoreinheit
- 17: Kohlenmonoxidableitung
- 18: Gasblase

## Patentansprüche

1. System zur extrakorporalen Elimination von Kohlenmonoxid, umfassend wenigstens eine Pumpe (5) und eine Gasaustauschkammer (6), wobei die wenigstens eine Pumpe (5) durch einen mit einer Kanüle (2) verbindbaren ersten Schlauchabschnitt (3) mit dem Blutkreislauf einer Person (1) verbindbar ist und über einen zweiten Schlauchabschnitt (4) mit der Gasaustauschkammer (6) verbunden ist, wobei das System dazu eingerichtet ist Blut mittels der wenigstens einen Pumpe (5) über den ersten Schlauchabschnitt (3) aus dem Blutkreislauf der Person (1) in die Gasaustauschkammer (6) abzuführen und aus der Gasaustauschkammer (6) über denselben ersten Schlauchabschnitt (3) in den Blutkreislauf der Person (1) zurückzuführen und wobei die Gasaustauschkammer (6) eine Sauerstoffzuführung (11) aufweist, die über eine Sauerstoffzuleitung (12) mit einer Sauerstoffquelle (13) verbunden ist und die Sauerstoffzuführung (11) ein Ausströmelement aufweist, das zur Bildung von Gasblasen eingerichtet ist,
**dadurch gekennzeichnet, dass**
a. durch die wenigstens eine Pumpe, insbesondere eine Schlauchpumpe, im deaktivierten nichtpumpenden Zustand ein Fluß zwischen dem ersten Schlauchabschnitt (3) und zweiten Schlauchabschnitt (4) verhindert ist, oder
b. der erste Schlauchabschnitt (3) oder der zweite Schlauchabschnitt (4) zumindest ein Ventil aufweist, mit dem ein Fluß zwischen dem Blutkreislauf der Person und der Gasaustauschkammer verhinderbar ist,
so dass die Gasaustauschkammer bei deaktivierter Pumpe oder mit den Fluß verhinderndem Ventil betreibbar und mit einem Druck über dem umgebenden Atmosphärendruck beaufschlagbar ist, ohne den Blutkreislauf der Person zu beeinflussen.

2. System nach Anspruch 1, **dadurch gekennzeichnet, dass** das System eine einzige Pumpe (5) aufweist, die bidirektional betreibbar ist oder der erste Schlauchabschnitt (3) mit zwei unidirektional betreibbaren Pumpen (5a, 5b) verbindbar ist, mit denen Blut im ersten Schlauchabschnitt (3) in entgegengesetzten Richtungen förderbar ist.

3. System nach einem der vorherigen Ansprüche, **dadurch gekennzeichnet, dass** zwischen dem zweiten Schlauchabschnitt (4) und der Gasaustauschkammer (6) ein Blasenfilter (7) angeordnet ist.

4. System nach Anspruch 3, **dadurch gekennzeichnet, dass** der Blasenfilter (7) Poren mit einem Durchmesser im Bereich von 5 µm bis 80 µm aufweist, besonders bevorzugt im Bereich von 5 µm bis 40 µm.

5. System nach einem der vorherigen Ansprüche, **dadurch gekennzeichnet, dass** die Sauerstoffquelle (13) eine Sauerstoffflasche ist.

6. System nach einem der vorherigen Ansprüche, **dadurch gekennzeichnet, dass** das Ausströmelement eine perforierte Leitung, eine perforierte Membran oder ein poröser Schaum ist.

7. System nach einem der vorherigen Ansprüche, **dadurch gekennzeichnet, dass** die Gasaustauschkammer (6) eine Kohlenmonoxidableitung (17) aufweist.

8. System nach Anspruch 13, **dadurch gekennzeichnet, dass** zwischen Gasaustauschkammer (6) und Kohlenmonoxidableitung (17) ein Schaumfilter (8) angeordnet ist.

9. System nach einem der vorherigen Ansprüche, **dadurch gekennzeichnet, dass** die Sauerstoffzuleitung (12) ein erstes Ventil (9) und die Kohlenmonoxidableitung (17) ein zweites Ventil (10) aufweist.

10. System nach einem der vorherigen Ansprüche, **dadurch gekennzeichnet, dass** die Gasaustauschkammer (6) eine erste Sensoreinheit (15) aufweist, mit der eine Kohlenmonoxidkonzentration bestimmbar ist.

11. System nach einem der vorherigen Ansprüche, **dadurch gekennzeichnet, dass** die Gasaustauschkammer (6) eine zweite Sensoreinheit (16) aufweist, mit der ein Druck innerhalb der Gasaustauschkammer (6) messbar ist.

12. System nach einem der vorherigen Ansprüche, **dadurch gekennzeichnet, dass** das System dazu eingerichtet ist Blut portionsweise zu behandeln, wobei es in einem ersten Betriebsmodus dazu eingerichtet ist Blut, insbesondere eine begrenzte Portion von Blut aus dem Blutkreislauf der Person (1) in die Gasaustauschkammer (6) zu befördern und in einem zweiten Betriebsmodus Blut in der Gasaustauschkammer (6) unter erhöhtem Druck mit Sauersoff in Kontakt zu bringen und Kohlenmonoxid zu eliminieren und in einem dritten Betriebsmodus Blut aus der Gasaustauschkammer (6) in den Blutkreislauf der Person (1) zurück zu befördern.

13. System nach einem der vorherigen Ansprüche, **dadurch gekennzeichnet, dass** das System eine Steuereinheit (14) aufweist, die dazu eingerichtet ist die Pumpe (5) oder die Pumpen (5a, 5b) und/oder nach Anspruch 9 das erste Ventil (9) zu steuern und/oder nach Anspruch 9 das zweite Ventil (10) zu steuern, insbesondere gemäß den Betriebsmodi des Anspruchs 12, und/oder nach Anspruch 10 die erste Sensoreinheit (15) auszulesen und/oder nach Anspruch 11 die zweite Sensoreinheit (16) auszulesen.

14. System nach einem der vorherigen Ansprüche, **dadurch gekennzeichnet, dass** das System in einem tragbaren Koffer angeordnet ist und mit diesem mobil transportierbar ist, wobei das System auch im Betrieb an einer Person mit der Person mobil transportierbar ist.

## Claims

1. System for extracorporeal elimination of carbon monoxide, comprising at least one pump (5) and a gas-exchange chamber (6), wherein the at least one pump (5) is connectable to the blood circuit of a person (1) by a first tube section (3), which is connectable to a cannula (2), and is connected to the gas-exchange chamber (6) via a second tube section (4), wherein the system is configured such that, by means of the at least one pump (5), it discharges blood into the gas-exchange chamber (6) from the blood circuit of the person (1) via the first tube section (3) and returns blood into the blood circuit of the person (1) from the gas-exchange chamber (6) via the same first tube section (3), and wherein the gas-exchange chamber (6) has an oxygen feed (11) which is connected to an oxygen source (13) via an oxygen-supply line (12), and the oxygen feed (11) has an outflow element which is configured for forming gas bubbles,
**characterized in that**
a. a flow between the first tube section (3) and the second tube section (4) is prevented by the at least one pump, in particular peristaltic pump, in the deactivated, non-pumping state, or
b. the first tube section (3) or the second tube section (4) has at least one valve by way of which a flow between the blood circuit of the person and the gas-exchange chamber is preventable,
so that the gas-exchange chamber is operable with pump deactivated or by way of the flow-preventing valve, and is subjectable to a pressure above the surrounding atmospheric pressure, without the blood circuit of the person being influenced.

2. System according to Claim 1, **characterized in that** the system has a single pump (5) which is operable in a bidirectional manner, or the first tube section (3) is connectable to two pumps (5a, 5b) which are operable in a unidirectional manner and by way of which blood in the first tube section (3) is deliverable in opposite directions.

3. System according to either of the preceding claims, **characterized in that** a bubble filter (7) is arranged between the second tube section (4) and the gas-exchange chamber (6).

4. System according to Claim 3, **characterized in that** the bubble filter (7) has pores with a diameter in the range from 5 um to 80 um, particularly preferably in the range from 5 um to 40 µm.

5. System according to one of the preceding claims, **characterized in that** the oxygen source (13) is an oxygen cylinder.

6. System according to one of the preceding claims, **characterized in that** the outflow element is a perforated line, a perforated membrane or a porous foam.

7. System according to one of the preceding claims, **characterized in that** the gas-exchange chamber (6) has a carbon-monoxide-discharge line (17).

8. System according to Claim 13, **characterized in that** a foam filter (8) is arranged between gas-exchange chamber (6) and carbon-monoxide-discharge line (17) .

9. System according to one of the preceding claims, **characterized in that** the oxygen-supply line (12) has a first valve (9), and the carbon-monoxide-discharge line (17) has a second valve (10).

10. System according to one of the preceding claims, **characterized in that** the gas-exchange chamber (6) has a first sensor unit (15) by way of which a carbon monoxide concentration is determinable.

11. System according to one of the preceding claims, **characterized in that** the gas-exchange chamber (6) has a second sensor unit (16) by way of which a pressure within the gas-exchange chamber (6) is measurable.

12. System according to one of the preceding claims, **characterized in that** the system is configured to treat blood in portions, wherein, in a first operating mode, said system is configured to convey blood, in particular a limited portion of blood, into the gas-exchange chamber (6) from the blood circuit of the person (1) and, in a second operating mode, said system is configured to bring blood in the gas-exchange chamber (6) into contact at elevated pressure with oxygen and to eliminate carbon monoxide and, in a third operating mode, said system is configured to convey blood back into the blood circuit of the person (1) from the gas-exchange chamber (6).

13. System according to one of the preceding claims, **characterized in that** the system has a control unit (14) which is configured to control the pump (5) or the pumps (5a, 5b) and/or, according to Claim 9, the first valve (9) and/or to control, according to Claim 9, the second valve (10), in particular according to the operating modes of Claim 12, and/or to read out, according to Claim 10, the first sensor unit (15) and/or to read out, according to Claim 11, the second sensor unit (16).

14. System according to one of the preceding claims, **characterized in that** the system is arranged in a portable case and is transportable in a mobile manner by way thereof, wherein, even during operation on a person, the system is transportable, along with the person, in a mobile manner.

## Revendications

1. Système d'élimination extracorporelle de monoxyde de carbone, comprenant au moins une pompe (5) et une chambre d'échange de gaz (6), l'au moins une pompe (5) pouvant être reliée à la circulation sanguine d'une personne (1) par le biais d'un premier tronçon de flexible (3) pouvant être relié à une canule (2) et étant reliée à la chambre d'échange de gaz (6) par l'intermédiaire d'un deuxième tronçon de flexible (4), le système étant conçu pour évacuer du sang à partir de la circulation sanguine de la personne (1) dans la chambre d'échange de gaz (6) par l'intermédiaire du premier tronçon de flexible (3) au moyen de l'au moins une pompe (5) et pour le renvoyer à partir de la chambre d'échange de gaz (6) par l'intermédiaire du même premier tronçon de flexible (3) dans la circulation sanguine de la personne (1) et la chambre d'échange de gaz (6) présentant une alimentation en oxygène (11) qui est reliée à une source d'oxygène (13) par l'intermédiaire d'une conduite d'amenée d'oxygène (12) et l'alimentation en oxygène (11) présentant un élément d'écoulement qui est conçu pour la formation de bulles de gaz,
**caractérisé en ce que**
a. par le biais de l'au moins une pompe, en particulier d'une pompe péristaltique, dans l'état désactivé non pompant, un flux entre le premier tronçon de flexible (3) et le deuxième tronçon de flexible (4) est empêché, ou
b. le premier tronçon de flexible (3) ou le deuxième tronçon de flexible (4) présente au moins une soupape, à l'aide de laquelle un flux entre la circulation sanguine de la personne et la chambre d'échange de gaz peut être empêché,
de sorte que la chambre d'échange de gaz puisse fonctionner lorsque la pompe est désactivée ou lorsque la soupape empêche le flux et puisse être soumise à une pression supérieure à la pression atmosphérique ambiante, sans influencer la circulation sanguine de la personne.

2. Système selon la revendication 1, **caractérisé en ce que** le système présente une seule pompe (5) qui peut fonctionner de manière bidirectionnelle ou le premier tronçon de flexible (3) peut être relié à deux pompes (5a, 5b) pouvant fonctionner de manière unidirectionnelle, pompes à l'aide desquelles du sang dans le premier tronçon de flexible (3) peut être refoulé dans des sens inverses.

3. Système selon l'une des revendications précédentes, **caractérisé en ce qu'**un filtre à bulles (7) est disposé entre le deuxième tronçon de flexible (4) et la chambre d'échange de gaz (6).

4. Système selon la revendication 3, **caractérisé en ce que** le filtre à bulles (7) présente des pores dotés d'un diamètre dans la plage de 5 um à 80 um, de manière particulièrement préférée dans la plage de 5 um à 40 µm.

5. Système selon l'une des revendications précédentes, **caractérisé en ce que** la source d'oxygène (13) est une bouteille d'oxygène.

6. Système selon l'une des revendications précédentes, **caractérisé en ce que** l'élément d'écoulement est une conduite perforée, une membrane perforée ou une mousse poreuse.

7. Système selon l'une des revendications précédentes, **caractérisé en ce que** la chambre d'échange de gaz (6) présente une conduite d'évacuation de monoxyde de carbone (17) .

8. Système selon la revendication 13, **caractérisé en ce qu'**un filtre en mousse (8) est disposé entre la chambre d'échange de gaz (6) et la conduite d'évacuation de monoxyde de carbone (17).

9. Système selon l'une des revendications précédentes, **caractérisé en ce que** la conduite d'amenée d'oxygène (12) présente une première soupape (9) et la conduite d'évacuation de monoxyde de carbone (17) présente une deuxième soupape (10).

10. Système selon l'une des revendications précédentes, **caractérisé en ce que** la chambre d'échange de gaz (6) présente une première unité de détection (15), à l'aide de laquelle une concentration de monoxyde de carbone peut être déterminée.

11. Système selon l'une des revendications précédentes, **caractérisé en ce que** la chambre d'échange de gaz (6) présente une deuxième unité de détection (16), à l'aide de laquelle une pression à l'intérieur de la chambre d'échange de gaz (6) peut être mesurée.

12. Système selon l'une des revendications précédentes, **caractérisé en ce que** le système est conçu pour traiter du sang par portions, celui-ci étant conçu, dans un premier mode de fonctionnement, pour transporter du sang, en particulier une portion limitée de sang, à partir de la circulation sanguine de la personne (1) dans la chambre d'échange de gaz (6) et, dans un deuxième mode de fonctionnement, pour amener du sang dans la chambre d'échange de gaz (6) en contact avec de l'oxygène sous une pression accrue et pour éliminer du monoxyde de carbone et, dans un troisième mode de fonctionnement, pour renvoyer du sang à partir de la chambre d'échange de gaz (6) dans la circulation sanguine de la personne (1) .

13. Système selon l'une des revendications précédentes, **caractérisé en ce que** le système présente une unité de commande (14) qui est conçue pour commander la pompe (5) ou les pompes (5a, 5b) et/ou la première soupape (9) selon la revendication 9 et/ou la deuxième soupape (10) selon la revendication 9, en particulier selon les modes de fonctionnement de la revendication 12, et/ou pour lire l'unité de détection (15) selon la revendication 10 et/ou pour lire la deuxième unité de détection (16) selon la revendication 11.

14. Système selon l'une des revendications précédentes, **caractérisé en ce que** le système est disposé dans une valise portable et peut être transporté de manière mobile à l'aide de celle-ci, le système pouvant être transporté de manière mobile par la personne même en cas de fonctionnement au niveau d'une personne.
